# EUROPEAN PATENT APPLICATION

(11) **EP 4 552 637 A1**
(43) Date of publication of application: **14.05.2025**
(21) Application number: 23835860.0
(22) Date of filing: 06.07.2023
(51) Int. Cl.: A61K 31/19, A61P 43/00, A61P 35/00

(54) **COMPOSITION FOR AMELIORATING OR TREATING CANCER CACHEXIA CONTAINING CALCIUM LACTATE AS ACTIVE INGREDIENT**

(30) Priority: 07.07.2022 KR 20220083857; 04.07.2023 KR 20230086516
(71) Applicant: Metimedi Pharmaceuticals Co., Ltd, Uiwang-si Gyeonggi-do 16006 (KR)
(72) Inventor: KIM, Hwan Mook, Seoul 06326 (KR)
(74) Representative: FRKelly
(86) International application number: PCT/KR2023/009578
(87) International publication number: WO 2024/010394

(57) **Abstract**

The present invention relates to a composition for ameliorating or treating cachexia containing calcium lactate as an active ingredient, and a method for treating cancer cachexia in a subject, the method comprising a step of administering calcium lactate or a pharmaceutically acceptable salt or solvate thereof to a subject in need thereof. The calcium lactate of the present invention acts as a Hif-1α inhibitor and exhibits an effect of suppressing body mass loss due to cancer, so that it can be used as a candidate substance for solving the problem of cancer cachexia and suggests a synergistic effect between a medical nutritional product focused on anorexia and malnutrition and a Hif-1α inhibitor.

## Description

### [Technical Field]

The present invention relates to a composition for ameliorating or treating cancer cachexia containing calcium lactate as an active ingredient and/or a method for treating cancer cachexia in a subject.

### [Background Art]

Cachexia is associated with various diseases such as heart failure, chronic obstructive pulmonary disease, chronic kidney disease, and HIV/AIDS, and is characterized by muscle loss, weight loss, fatigue, and malnutrition. Among these, cancer cachexia is a complex metabolic syndrome accompanying malignant tumors and is defined as a body weight loss of more than 5% due to muscle and fat loss within 6 months of the onset of cancer. Cachexia can occur in various medical conditions, but cancer cachexia is closely related to terminal cancer. Approximately 50% of all cancer patients, and especially about 80% of terminal cancer patients, are affected by cancer cachexia, and suffer from a decreased quality of life and increased mortality due to muscle and fat loss. Cancer cachexia is characterized by weight loss due to significant loss of muscle and adipose tissue and has different characteristics from muscle and fat loss through appetite suppression. Diagnostic criteria for cachexia include weight loss greater than 5%, elevated serum levels of inflammatory markers (IL6, CRP1, TNFα), decreased exercise ability, and anorexia.

Treatments for cancer cachexia include non-steroidal anti-inflammatory drugs (NSAIDs), β2-adrenergic agonists, corticosteroids, and ghrelin. The most used drugs for cancer cachexia currently on the market are COX-2 inhibitors and TNF-α inhibitors. COX-2 inhibitors are drugs that reduce skeletal muscle loss by inhibiting COX-2, which is involved in the production of prostaglandins, inflammatory substances produced in large quantities in cancer tissue. Celecoxib is commercially available as a COX-2 inhibitor. However, celecoxib has been reported to have side effects such as anemia, gastric ulcers, allergies, heart attacks, and strokes.

TNF-α inhibitors are drugs that kill cancer cells by reducing the expression of TNF-α, which is highly expressed in the serum of cancer cachexia patients. Thalidomide is sold on the market as a TNF-α inhibitor. However, these drugs are not effective as a cancer cachexia treatment, and have side effects such as depression, heart failure, dyspnea, vomiting, rash, hypertension, and birth defects during pregnancy. Most of the drugs currently under development or on the market have many side effects and lack evidence for therapeutic effects. Therefore, the development of cancer cachexia treatment with a new mechanism is urgently needed.

### [Prior patent literature]

Republic of Korea Patent Publication Number 10-2019-0103985

### [Disclosure]

### [Technical Problem]

The object of the present invention is to provide a novel composition for ameliorating or treating cancerous cachexia.

Another object of the present invention is to provide a novel method for ameliorating or treating cancerous cachexia.

### [Technical Solution]

To achieve the above object, the present invention provides a composition for ameliorating or treating cachexia containing calcium lactate as an effective ingredient.

In one embodiment of the present invention, the cachexia is preferably caused by cancer but is not limited thereto.

In another embodiment of the present invention, the composition is preferably for ameliorating at least one symptom selected from the group consisting of decreased appetite, weight loss, increased fatigue, muscle loss, fat loss, and hematopoietic toxicity, but is not limited thereto.

The present invention also provides a method for treating cancerous cachexia in a subject,
the method comprising administering calcium lactate or a pharmaceutically acceptable salt or solvate thereof to a subject in need thereof.

The present invention also provides a use of calcium lactate in the manufacture of a medicament for preventing or treating cancerous cachexia in a subject.

The present invention is described below with the results of the following examples together with drawings.

Figure 1 is a drawing showing that Hif-1α inhibitors suppress body mass loss caused by cancer. The important involvement of Hif-1α in cancer cachexia suggests a synergistic effect between medical nutrition products focusing on anorexia and malnutrition and Hif-1α inhibitors.

Anoxia/hypoxia of the TME significantly affects various aspects of cancer cachexia and worsens the patient's condition, and medical nutrition products and the Hif-1α inhibitor of the present invention have a common goal of solving the problem of cachexia with a complementary approach.

Figures 2 to 4 are drawings showing that the calcium lactate of the present invention degrades tumor Hif-1α protein under hypoxic conditions without affecting normal cells.

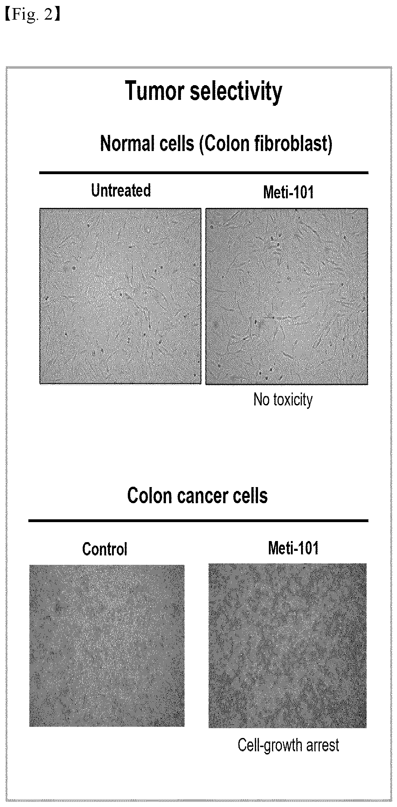

The upper panel of Figure 2 confirms the effect of calcium lactate treatment on survival in normal colon fibroblasts, indicating that calcium lactate treatment does not cause toxicity to normal cells and thus does not change survival.

The lower panel of Figure 2 confirms the effect of calcium lactate treatment on survival in colorectal cancer cells, and when calcium lactate is treated, cancer cell death can be observed due to the anticancer effect.

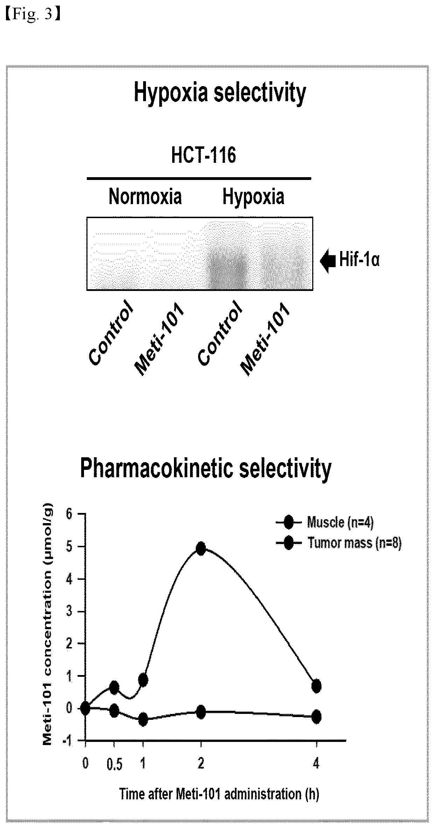

The upper panel of Fig. 3 shows the change in the expression of Hif-1α protein after calcium lactate treatment in colon cancer cells under normoxia or hypoxia conditions. Hif-1α expression cannot be observed under normoxia conditions and that the expression of Hif-1α expressed under hypoxia conditions is reduced by calcium lactate treatment.

The lower panel of Fig. 3 shows the PK analysis in muscle and tumor tissues after calcium lactate treatment. The concentration of lactate increases in tumor tissues 2 hours after calcium lactate administration, but there is no change in muscle tissue.

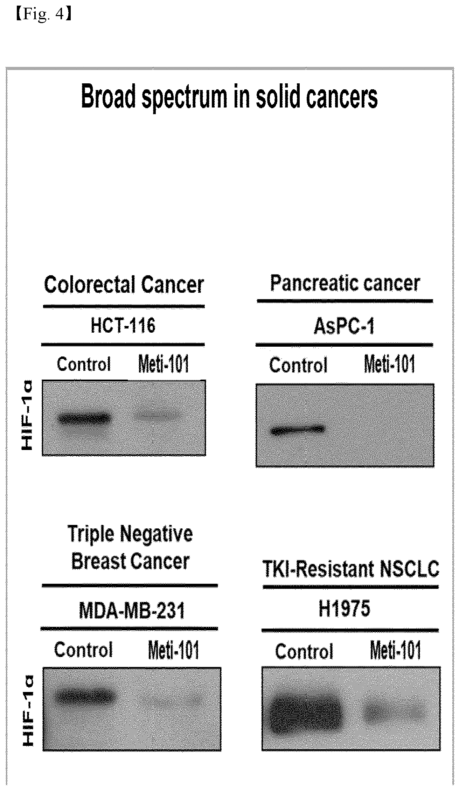

Fig. 4 shows the change in Hif-1α expression due to calcium lactate treatment in HCT-116 (colon cancer), AsPC-1 (pancreatic cancer), MDA-MB-231 (breast cancer), and H1975 (lung cancer) cell lines. The expression of Hif-1α is reduced after calcium lactate treatment in all cell lines.

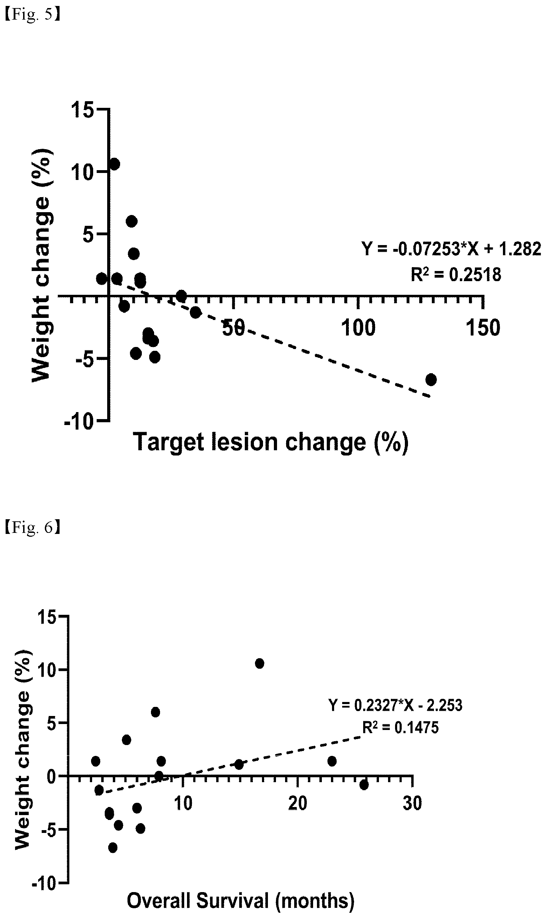

Figures 5 and 6 are drawings showing the results of the phase 2 clinical trial. As can be seen in Figure 5, weight change and tumor size change have a negative correlation. Among a total of 16 patients with metastatic colorectal cancer refractory to standard therapy, 8 patients maintained or increased their weight, and the increase ranged from 0.0 to 10.6%, 6 patients decreased their weight, and the decrease ranged from 0.8 to 6.7%. Cachexia was observed in 2 patients. The median increase in tumor size was 17% in 8 patients with weight loss including cachexia, and 9.5% in 8 patients with weight maintenance or increase.

As shown in Figure 6, there is a positive correlation between weight change and overall survival. The median overall survival was 4.15 months in 8 patients with weight loss including cachexia, and 8 months in 8 patients with maintained or increased weight. Only 2 out of 16 patients progressed to cancerous cachexia, suggesting that calcium lactate can prevent patients from cachexia with an efficacy of 87.5%, and the drug efficacy of calcium lactate for tumor growth arrest was 68.8%, showing the highest potential in the metastatic stage of antitumor effect. Non-DLT (dose limiting toxicity) was observed in phase 2 clinical trials.

### Pharmaceutical composition for preventing or treating cachexia

The present invention provides a pharmaceutical composition for preventing or treating cachexia comprising calcium lactate or a pharmaceutically acceptable salt thereof.

The term 'cachexia' used in this specification refers to a severe systemic weakness that can be seen in the terminal stages of cancer, tuberculosis, diabetes, acquired immunodeficiency syndrome (AIDS), etc., and frequently occurs in patients with gastrointestinal cancers such as gastric cancer, esophageal cancer, pancreatic cancer, and colon cancer, and lung cancer. It shows symptoms such as decreased appetite, loss of weight and physical strength due to loss of muscle and fat, anemia, lethargy, and indigestion, and it shows a state in which it is difficult to eat normally due to decreased appetite, or a state in which weight is lost even if food is eaten normally. When cachexia occurs, the patient shows a low response to anticancer chemotherapy or radiotherapy, which lowers the quality of life of the patient, shortens life expectancy, and causes death due to weight loss in 10 to 20% of all cancer patients.

In the pharmaceutical composition according to the present invention, the cachexia may be caused by cancer. In one embodiment of the present invention, the cachexia caused by cancer is expressed as 'cancer cachexia'.

Cancer as defined herein may include colorectal cancer, leukemia, lymphoma, myeloma, myelodysplastic syndrome, breast cancer, head and neck cancer, esophageal cancer, stomach cancer, colon cancer, rectal cancer, anal cancer, hepatocellular carcinoma, cholangiocarcinoma, gallbladder cancer, pancreatic cancer, lung cancer (non-small cell lung cancer, small cell lung cancer), thymic cancer, kidney cancer, bladder cancer, prostate cancer, testicular cancer, ovarian cancer, cervical cancer, sarcoma, gastrointestinal stromal tumor (GIST), cancer of unknown primary site, mesothelioma, melanoma, neuroendocrine tumor, skin cancer, blood cancer, etc.

The pharmaceutical composition according to the present invention may ameliorate at least one symptom selected from the group consisting of decreased appetite, weight loss, increased fatigue, muscle loss, fat loss, and hematopoietic toxicity, and preferably may ameliorate at least one symptom selected from the group consisting of weight loss, muscle loss, and fat loss.

In the pharmaceutical composition according to the present invention, the term 'pharmaceutically acceptable salt' means any organic or inorganic addition salt of calcium lactate having a concentration that is relatively nontoxic and harmless to the patient and has an effective effect, and the side effects caused by the salt do not reduce the beneficial effects of calcium lactate. These salts can use an inorganic acid and an organic acid as the free acid, and the inorganic acid can be hydrochloric acid, hydrobromic acid, nitric acid, sulfuric acid, perchloric acid, phosphoric acid, etc., and the organic acid can be citric acid, acetic acid, lactic acid, maleic acid, fumaric acid, gluconic acid, methanesulfonic acid, glycolic acid, succinic acid, tartaric acid, galacturonic acid, embonic acid, glutamic acid, aspartic acid, oxalic acid, (D) or (L) malic acid, methanesulfonic acid, ethanesulfonic acid, 4-toluenesulfonic acid, salicylic acid, benzoic acid, malonic acid, etc. Additionally, these salts include alkali metal salts (sodium salts, potassium salts, etc.) and alkaline earth metal salts (calcium salts, magnesium salts, etc.). For example, acid addition salts include acetate, aspartate, benzate, besylate, bicarbonate/carbonate, bisulfate/sulfate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methyl sulfate, naphthylate, 2-naphthylate, nicotinate, nitrate, orotate, oxalate, palmitate, pamoate, phosphate/hydrogen phosphate/dihydrogen phosphate, saccharate, stearate, It may contain succinate, tartrate, tosylate, trifluoroacetate, aluminum, arginine, benzathine, calcium, choline, diethylamine, diolamine, glycine, lysine, magnesium, meglumine, olamine, potassium, sodium, tromethamine, and zinc salts.

The acid addition salt according to the present invention can be prepared by a conventional method, for example, dissolving calcium lactate in an organic solvent, for example, methanol, ethanol, acetone, methylene chloride, acetonitrile, etc., adding an organic acid or inorganic acid, and filtering and drying the resulting precipitate, or by distilling the solvent and an excess acid under reduced pressure and then drying or crystallizing the same in the presence of an organic solvent.

In addition, a pharmaceutically acceptable metal salt can be prepared using a base. An alkali metal or alkaline earth metal salt is obtained, for example, by dissolving a compound in an excess of an alkali metal hydroxide or an alkaline earth metal hydroxide solution, filtering the undissolved compound salt, and evaporating and drying the filtrate. At this time, it is pharmaceutically suitable to prepare a sodium, potassium or calcium salt as the metal salt. **In** addition, the corresponding silver salt is obtained by reacting an alkali metal or alkaline earth metal salt with a suitable silver salt (e.g., silver nitrate).

Furthermore, the present invention may comprise not only the calcium lactate and its pharmaceutically acceptable salts, but also all possible solvates, hydrates, isomers, optical isomers, etc. that can be prepared therefrom.

The pharmaceutical composition according to the present invention may be formulated for oral administration, intramuscular administration, intravenous administration, intraperitoneal administration, subcutaneous administration, intradermal administration, or topical administration.

The calcium lactate of the present invention or a pharmaceutically acceptable salt thereof can be administered in various oral and parenteral dosage forms during clinical administration, and when formulated, it is manufactured using diluents or excipients such as fillers, bulking agents, binders, wetting agents, disintegrants, and surfactants that are commonly used.

Solid preparations for oral administration include tablets, patients, powders, granules, capsules, troches, etc., and these solid preparations are prepared by mixing one or more of the calcium lactate of the present invention or a pharmaceutically acceptable salt thereof with at least one excipient, such as starch, calcium carbonate, sucrose, lactose, or gelatin. **In** addition to simple excipients, lubricants such as magnesium stearate and talc are also used. Liquid preparations for oral administration include suspensions, solutions, emulsions, and syrups, and in addition to commonly used simple diluents such as water and liquid paraffin, they may contain various excipients such as wetting agents, sweeteners, flavoring agents, and preservatives.

Preparations for parenteral administration include sterile aqueous solutions, non-aqueous solutions, suspensions, emulsions, lyophilized preparations, suppositories, etc. Non-aqueous solutions and suspensions may include propylene glycol, polyethylene glycol, vegetable oils such as olive oil, and injectable esters such as ethyl oleate. Suppository bases may include witepsol, macrogol, tween 61, cacao butter, laurin butter, glycerol, gelatin, etc.

The pharmaceutical composition may further include a carrier, excipient, or diluent. The carrier, excipient, or diluent can include, for example, lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, or mineral oil.

In addition, the pharmaceutical composition may further comprise a known effective ingredient having an activity for preventing or treating cancerous cachexia. An effective dosage for the human body of the calcium lactate of the present invention or a pharmaceutically acceptable salt thereof may vary depending on the patient's age, body weight, gender, dosage form, health condition, and disease severity, and is generally about 0.001 to 100 mg/kg/day, and preferably 0.01 to 35 mg/kg/day. When based on an adult patient weighing 70 kg, the dosage is generally 0.07 to 7000 mg/day, and preferably 0.7 to 2500 mg/day, and may be administered once or several times a day at regular intervals depending on the judgment of a doctor or pharmacist.

### Health functional food and health food composition for ameliorating cachexia

In addition, the present invention provides a health functional food composition for ameliorating cachexia containing calcium lactate or a food-based acceptable salt thereof.

The term "health functional food" used in the present invention refers to a food manufactured and processed in the form of tablets, capsules, pills, liquids, powders, granules, etc. using raw materials or ingredients having useful functionality for the human body. Here, "functionality" means obtaining a useful effect for health purposes such as regulating nutrients for the structure and function of the human body or physiological effects. The health functional food of the present invention can be manufactured by a method commonly used in the art and can be manufactured by adding raw materials and ingredients commonly added in the art during the manufacturing process. In addition, the formulation of the health functional food can be manufactured without limitation if it is a formulation recognized as a health functional food. The health functional food composition of the present invention can be manufactured into various forms of formulations, and unlike general drugs, it has the advantage of having no side effects that may occur with long-term use of drugs since it uses food as a raw material , and is highly portable, so the health functional food of the present invention can be taken as a supplement to enhance the effects of a cancer cachexia treatment agent or an anticancer agent.

Furthermore, the present invention provides a health functional food composition for ameliorating cachexia, which comprises calcium lactate or a food-based acceptable salt thereof.

The health functional food composition or health food composition according to the present invention can add the calcium lactate or a food-based acceptable salt thereof to health functional foods or health foods such as foods, beverages, etc. for the purpose of preventing or ameliorating cancerous cachexia.

There is no limitation on the type of the food. Examples of foods to which the calcium lactate or a food-based acceptable salt thereof according to the present invention can be added include drinks, meat, sausages, bread, biscuits, rice cakes, chocolate, candies, snacks, confectionery, pizza, ramen, other noodles, gums, dairy products including ice cream, various soups, beverages, alcoholic beverages, vitamin complexes, dairy products, and dairy products, and comprises all health foods and health functional foods in the conventional sense.

The health functional food and health food composition according to the present invention can be added to food as it is or used together with other food or food ingredients and can be used appropriately according to a conventional method. The mixing amount of the calcium lactate or its food based acceptable salt according to the present invention can be appropriately determined depending on its purpose of use (prevention or ameliorate). Generally, the amount of the calcium lactate or its food based acceptable salt in the health food and health functional food can be added in an amount of 0.1 to 90 parts by weight of the total food weight. However, in the case of long-term intake for the purpose of maintaining health or regulating health, the amount can be below the above range, and since there is no problem in terms of safety, the active ingredient can also be used in an amount above the above range.

The health food and health functional food composition of the present invention comprises calcium lactate or a food-based acceptable salt thereof according to the present invention as an essential ingredient in the indicated ratio, and has no particular limitations on other ingredients, and may contain various flavoring agents or natural carbohydrates as additional ingredients like conventional beverages. Examples of the above-mentioned natural carbohydrates are conventional sugars such as monosaccharides, for example, glucose, fructose, etc.; disaccharides, for example, maltose, sucrose, etc.; and polysaccharides, for example, dextrin, cyclodextrin, etc., and sugar alcohols such as xylitol, sorbitol, and erythritol. As flavoring agents other than those described above, natural flavoring agents (thaumatin, stevia extract (e.g., rebaudioside A, glycyrrhizin, etc.)) and synthetic flavoring agents (saccharin, aspartame, etc.) can be advantageously used. The proportion of the natural carbohydrate is generally about 1 to 20 g, preferably about 5 to 12 g, per 100 g of the health functional food composition of the present invention.

In addition to the above, the health food and health functional food composition containing calcium lactate or a food-based acceptable salt thereof according to the present invention may comprise various nutrients, vitamins, minerals (electrolytes), flavoring agents such as synthetic flavoring agents and natural flavoring agents, coloring agents and thickeners (cheese, chocolate, etc.), pectic acid and its salts, alginic acid and its salts, organic acids, protective colloid thickeners, pH regulators, stabilizers, preservatives, glycerin, alcohol, carbonating agents used in carbonated beverages, etc. In addition, the health food and health functional food composition of the present invention may contain fruit pulp to produce natural fruit juice and fruit juice drinks and vegetable drinks. These ingredients may be used independently or in combination. The ratio of these additives is not limited thereto but is generally selected in the range of 0.1 to about 20 parts by weight per 100 parts by weight of the health food and health functional food composition comprising the effective substance of the present invention.

### Anticancer adjuvant composition

In addition, the present invention provides an anticancer adjuvant composition comprising the pharmaceutical composition for preventing or treating cachexia.

The term "anticancer adjuvant" used in the present invention can be used to enhance the anticancer treatment effect and suppress or ameliorate the side effects of an anticancer agent and can be administered to a patient in combination with an anticancer agent.

The anticancer adjuvant composition of the present invention can exhibit an effect of suppressing or ameliorating cachexia symptoms such as muscle loss, weight loss, fat loss, hematopoietic toxicity, and appetite loss caused by cancer, thereby enhancing the anticancer effect of an anticancer agent.
he existing anticancer agents defined herein may include cyclophosphamide, methotrexate, 5-fluorouracil, doxorubicin, mustine, vincristine, procarbazine, prednisolone, bleomycin, vinblastine, dacarbazine, etoposide, cisplatin, epirubicin, cisplatin, capecitabine, oxaliplatin, and immunotherapy anticancer agents.

The above anticancer adjuvant composition may additionally comprise one or more active ingredients having the same or similar function in addition to calcium lactate or a pharmaceutically acceptable salt thereof as an active ingredient. The above anticancer adjuvant composition may be administered orally or parenterally at the time of clinical administration, and may be administered by intraperitoneal injection, rectal injection, subcutaneous injection, intravenous injection, intramuscular injection, intrauterine epidural injection, intracerebrovascular injection, or intrathoracic injection at the time of parenteral administration, and may be used in the form of a general pharmaceutical preparation.

The above anticancer adjuvant can be used alone or in combination with methods using surgery, radiotherapy, hormone therapy, chemotherapy, and biological response modifiers. The daily dosage of the above anticancer adjuvant is about 0.0001 to 1000 mg/kg, preferably 1 to 100 mg/kg, and is preferably administered once a day or several times divided, but the range varies depending on the patient's weight, age, gender, health condition, diet, administration time, administration method, excretion rate, and disease severity. The anticancer adjuvant of the present invention can be administered in various parenteral formulations during actual clinical administration, and when formulated, it is prepared using diluents or excipients such as commonly used fillers, extenders, binders, wetting agents, disintegrants, and surfactants. Formulations for parenteral administration include sterile aqueous solutions, non-aqueous solvents, suspensions, emulsions, lyophilized preparations, and suppositories. Non-aqueous solvents and suspending agents can be used, such as propylene glycol, polyethylene glycol, vegetable oils such as olive oil, and injectable esters such as ethyl oleate. Suppository bases can be used, such as witepsol, macrogol, Tween 61, cacao butter, laurin butter, and glycerogelatin.

### [Advantageous Effects]

According to the present invention, the calcium lactate of the present invention acts as a Hif-1α inhibitor and exhibits an effect of suppressing body mass loss due to cancer, so that it can be used as a candidate substance for solving the problem of cancer cachexia, and suggests a synergistic effect between a medical nutritional product focusing on anorexia and malnutrition and a Hif-1α inhibitor.

### [Description of Drawings]

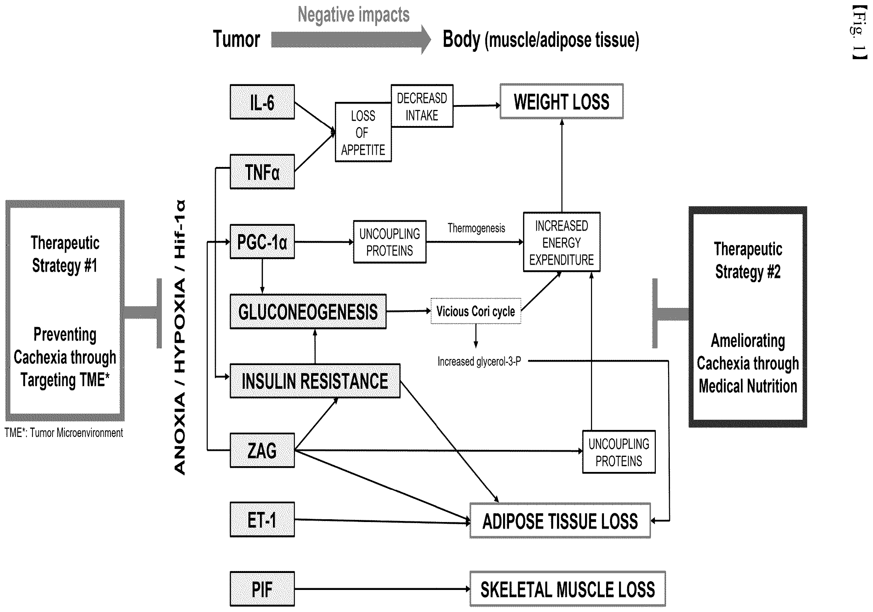

Figure 1 is a drawing showing that Hif-1α inhibitors suppress body mass loss caused by cancer. The important involvement of Hif-1α in cancer cachexia suggests a synergistic effect between medical nutrition products focusing on anorexia and malnutrition and Hif-1α inhibitors.

Anoxia/hypoxia of TME significantly affects various aspects of cancer cachexia and worsens the patient's condition, and the Hif-1α inhibitors and medical nutrition products of the present invention have a common goal of solving the problem of cachexia with a complementary approach.

Figures 2 to 4 are drawings showing that the calcium lactate of the present invention degrades tumor Hif-1α protein under hypoxic conditions without affecting normal cells. The upper panel of Figure 2 confirms the effect of calcium lactate treatment on survival in normal colon fibroblasts, the lower panel of Figure 2 confirms the effect of calcium lactate treatment on survival in colorectal cancer cells, and the upper panel of Figure 3 confirms the change in expression of Hif-1α protein after calcium lactate treatment in colon cancer cells under normoxia or hypoxia conditions, and the lower panel of Figure 3 shows PK analysis in muscle and tumor tissue after calcium lactate treatment.

Figure 4 shows the change in Hif-1α expression by calcium lactate treatment in HCT-116 (colon cancer), AsPC-1 (pancreatic cancer), MDA-MB-231 (breast cancer), and H1975 (lung cancer) cell lines.

In Figures 2 to 4, Meti-101 means calcium lactate.

Figures 5 to 6 are pictures showing the results of phase 2 clinical trials.

As can be seen in Figure 5, weight change and tumor size change have a negative correlation. Among a total of 16 metastatic colon cancer patient's refractory to standard therapy, 8 maintained or increased their weight, and the increase was 0.0 to 10.6%, 6 decreased their weight, and the weight decrease was 0.8 to 6.7%, and cachexia was observed in 2 patients. The median increase in tumor size was 17% in the 8 patients who lost weight including cachexia and 9.5% in the 8 patients who maintained or gained weight.

As shown in Figure 6, there is a positive correlation between weight change and overall survival. The median overall survival was 4.15 months in 8 patients with weight loss including cachexia, and 8 months in 8 patients with maintained or increased weight. Only 2 out of 16 patients progressed to cancerous cachexia, suggesting that calcium lactate can prevent patients from cachexia with an efficacy of 87.5%, and the drug efficacy of calcium lactate for tumor growth arrest was 68.8%, showing the highest potential in the metastatic stage of antitumor effect. Non-DLT (dose limiting toxicity) was observed in phase 2 clinical trials.

### [Mode for Invention]

Hereinafter, the present invention will be described in more detail through examples, but these are merely illustrative and are not intended to limit the scope of the present invention. It is obvious to those skilled in the art that the embodiments described below can be modified without departing from the essential gist of the invention.

### Example 1: Cytotoxicity assay

5x10³ of normal colon fibroblast cell line CCD-18Co or colon cancer cell line HCT-116 were cultured in a 96-well plate at 37°C, 5% CO₂. Incubator and observed under a microscope after 48 hours.

### Example 2: Western blot

Tumor tissue or HCT-116 and AsPC-1 (pancreatic cancer cell), MDA-MB-231 (breast cancer cell), H1975 (lung cancer cell) were cultured in a 24-well plate under hypoxia 1% O₂ conditions, and proteins from the untreated and treated groups of calcium lactate were extracted, and then transferred to polyvinylidene difluoride membranes after electrophoresis. Then, Hif-1α antibody was diluted in TBST and attached to the protein on the membrane, washed with TBST, and treated with secondary antibody, and the band results were confirmed using X-ray film or Gel doc equipment.

### Example 3: Tissue pharmacokinetics (PK)

Balb/c nude mice were obtained and 1x10⁶ HCT-116 was implanted subcutaneously to grow tumors. After 20 mg/kg of calcium lactate was injected S.C., muscle tissue and tumor tissue were obtained before administration and 30 minutes, 1, 2, and 4 hours after administration. Each obtained tissue was analyzed for changes in lactate concentration over time using a lactate analysis kit.

### Example 4: Efficacy of Calcium Lactate for Cachexia in the Clinical Trial

The clinical study was a phase II clinical trial targeting patients with metastatic colorectal cancer refractory to standard therapy.

Twenty-two patients were enrolled, and 16 completed the trial (see Table 1).

The change in body weight was calculated as the change from the value measured at screening to the value after 1 month of calcium lactate treatment, and the change in tumor size of the target lesion was measured based on RECIST 1.1. The administration method was subcutaneous injection, and the administration dose was 100 mg/day. One cycle consisted of 4 weeks of treatment with 5 days of I.P. administration subcutaneously once daily followed by 2 days of rest.

Tumor assessment was performed every 2 cycles compared to baseline.

The clinical outcomes were the primary outcome of disease control rate, and the secondary outcomes were overall survival, progression-free survival, and safety. Body weight was measured 1 month after the start of administration and expressed as the increase or decrease in body weight before and after administration. Tumor response was performed with a CT scan 2 months after the start of administration and expressed as the increase or decrease in tumor size before and after administration. SD means that tumor growth has stopped, and PD means that the tumor has increased by 20% or more.

**[TABLE 1]**

| **#** | **Screening no.** | **BMI** | **Weight T₀ (kg)** | **Weight T₁ₘₒ (kg)** | **Weight change** | **SLD change at target lesions** | **Overall tumor response** | **Overall survival (months)** |
|---|---|---|---|---|---|---|---|---|
| 1 | S01-017 | 17.7 | 50 | 51.7 | 3.4% | 10.0% | PD | 5.1 |
| 2 | S01-016 | 18,9 | 55.3 | 52.6 | -4.9% | 18.4% | SD | 6.3 |
| 3 | S01-011 | 21.5 | 62.4 | 69 | 10.6% | 2.2% | SD | 16.7 |
| 4 | S02-002 | 21.9 | 60.5 | 60 | -0.8% | 6.2% | SD | 25.8 |
| 5 | S01-006 | 22.2 | 62.1 | 63 | 1.4% | 12.5% | SD | 2.4 |
| 6 | S01-014 | 22.3 | 65.2 | 62.2 | -4.6% | 10.8% | SD | 4.4 |
| 7 | S02-007 | 22.6 | 67 | 65 | -3.0% | 15.7% | SD | 6 |
| 8 | S01-002 | 22.9 | 56.2 | 56.8 | 1.1% | 12.6% | SD | 14.9 |
| 9 | S01-008 | 24.0 | 65 | 68.9 | 6,0% | 9.1% | SD | 7.6 |
| 10 | S01-010 | 24.9 | 69.1 | 68.2 | -1.3% | 34.7% | PD | 2.7 |
| 11 | S02-003 | 25.3 | 67.5 | 65.1 | -3.6% | 17.7% | SD | 3.6 |
| 12 | S01-007 | 26.0 | 68.8 | 68.8 | 0.0% | 29.0% | PD | 7.9 |
| 13 | S02-005 | 27.1 | 73 | 74 | 1.4% | -2.9% | SD | 23 |
| 14 | S02-009 | 27.4 | 94.4 | 88.1 | -6.7% | 129.2% | PD | 3.9 |
| 15 | S01-015 | 28.1 | 84.8 | 86 | 1.4% | 3.3% | PD | 8.1 |
| 16 | S02-006 | 35.3 | 90.6 | 87.5 | -3.4% | 15.6% | SD | 3.6 |

Table 1 shows the characteristics of the clinical study subjects.

BMI and Weight T0 were measured at the screening visit, and Weight T1mo and SLD changes in the target lesion were measured 1 and 2 months after IP administration, respectively. SLD is an abbreviation for sum of longest diameters. SD and PD are abbreviations for stable disease and progressive disease, respectively.

Since 11 out of 16 patients had stable disease, the disease control rate was 68.75%.

**[TABLE 2]**

| **Characteristics** | **Number (%)** | **Comments** |
|---|---|---|
| Weight gain | 8 (50.0) | |
| weight loss | 6 (37.5) | |
| Cachexia | 2 (12.5) | S01-016, S02-009 |
| Total | 16 (100.0) | |

Table 2 summarizes the weight changes in the clinical trial. Cancer cachexia is defined as an unintended weight loss of 5% or more within 6 months of cancer onset and a BMI < 20 and weight loss > 2%. As shown in the table, among the 16 patients who completed the clinical trial, 8 patients had weight gain after calcium lactate administration, 6 patients had weight loss (not corresponding to cachexia), and 2 patients had cachexia.

## Claims

1. A composition for ameliorating or treating cachexia containing calcium lactate as an active ingredient.

2. The composition of claim 1, wherein the cachexia is caused by cancer.

3. The composition of claim 1, wherein the composition ameliorates at least one symptom selected from the group consisting of decreased appetite, weight loss, increased fatigue, muscle loss, fat loss, and hematopoietic toxicity.

4. A method for treating cancerous cachexia in a subject,
the method comprising a step of administering calcium lactate or a pharmaceutically acceptable salt or solvate thereof to a subject in need thereof.

5. Use of calcium lactate in the manufacture of a medicament for preventing or treating cancerous cachexia in a subject.
